(19) Europäisches Patentamt
European Patent Office
Office européen des brevets

(11) EP 2 444 815 A2

(12) **EUROPEAN PATENT APPLICATION**
published in accordance with Art. 153(4) EPC

(43) Date of publication:
**25.04.2012 Bulletin 2012/17**

(21) Application number: **10789702.7**

(22) Date of filing: **15.06.2010**

(51) Int Cl.:
***G01N 33/68*** *(2006.01)* ***C07D 405/12*** *(2006.01)*
***G01N 33/533*** *(2006.01)* ***A61K 8/64*** *(2006.01)*

(86) International application number:
**PCT/KR2010/003853**

(87) International publication number:
**WO 2010/147375 (23.12.2010 Gazette 2010/51)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **17.06.2009 KR 20090054085**

(71) Applicant: **Innopharmascreen Inc.**
**Asan-si, Chungcheongnam-do 336-851 (KR)**

(72) Inventors:
- **KANG, In-Cheol**
  **Seoul 135-796 (KR)**
- **PARK, Chan-Won**
  **Suwon-si**
  **Gyeonggi-do 441-749 (KR)**

(74) Representative: **Richter Werdermann Gerbaulet Hofmann**
**Patentanwälte**
**Neuer Wall 10**
**20354 Hamburg (DE)**

(54) **METHOD FOR SCREENING FOR SUBSTANCE WHICH INHIBITS INTERACTION BETWEEN BETA AMYLOID PEPTIDE AND VEGF AND INHIBITOR SEARCHED BY SAME**

(57) There is provided a compound found by screening a material for inhibiting a binding between a beta-amyloid 1-42 peptide and VEGF165, in which the inhibition material screened according to the present invention can improve effectiveness as a material for treating Alzheimer's disease.

[F[[FIG. 5]

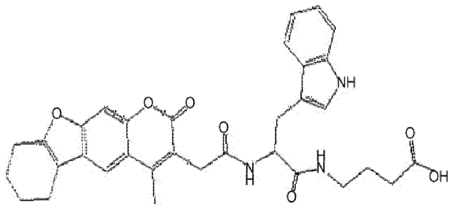

(IPS-04001) : 4-{3-(1*H*-Indol-3-yl)-2-[2-(6-methyl-8-oxo-1,2,3,4-tetrahydro-8*H*-9,11-dioxa-benzo[*b*]flioren-7-yl)-acetylamino]-propionylamino}-butyric acid

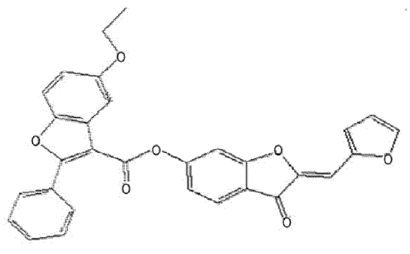

(IPS-04002) : 5-Ethoxy-2-phenyl-benzofuran-3-carboxylic acid 2-furan-2-ylmethylene-3-oxo-2,3-dihydro-benzofyran-6-ylester

EP 2 444 815 A2

## Description

### CROSS-REFERENCE TO RELATED APPLICATIONS

This application claims the priority of Korean Patent Application No. 10-2009-0054085 filed on June 17, 2009, in the Korean Intellectual Property Office, the disclosure of which is incorporated herein by reference.

### BACKGROUND OF THE INVENTION

#### Field of the Invention

The present invention relates to a compound that is found out by screening a material for inhibiting a binding of a beta-amyloid 1-42 peptide and VEGF165. The inhibition material screened according to the present invention can increase effectiveness as a material for treating Alzheimer's disease.

#### Description of the Related Art

Alzheimer's disease to be a major cause of dementia is characterized by loss of memory, a thinking decrease, a progressive neurodegeneration, and the like. Pathologic features of Alzheimer's disease may be neurofibrillary tangles shown in a cell and senile plaques accumulated outside neurocyte. All of Familial Alzheimer's disease (FAD) and Sporadic Alzheimer's disease (SAD) exhibit the above pathologic features. It was revealed that a major component of the senile plaques among them is a toxic protein called a beta-amyloid (Aβ) and it was reported that excess accumulation of the beta-amyloid is a common form (Parihar MS, Hemnani T., J Clin Neurosci. 2004, Jun;11(5):456-67, Selkoe DJ., Nature. 1999 Jun 24;399 (6738 Suppl):A23-31).

The amyloid has the structure of β pleated sheet thereby calling the beta-amyloid, and is generally composed of 39~43 amino acids. Additionally, an aqueous peptide forms β-pleated sheet formation to form a fibril so that it is easily precipitated to be toxic. It is thought that the above toxicity is a major cause of Alzheimer's disease (AD). APP695, APP751, and APP770 that are a precursor protein with high molecular weight are a metabolite produced by degradation through a metabolic process by a protease.

APP that is an integral membrane protein is metabolized in two pathways, such as an amyloidogenic pathway and a non-amyloidogenic pathway. The amyloidogenic pathway produces the beta-amyloid by a protease, such as a beta-secretase and a gamma-secretase, and at this point, concomitantly an extracellular domain is cleaved to secrete out of cell in a type of APP β. On the contrary, the non-amyloidogenic pathway is superior in a normal person and does not produce the beta-amyloid, in which 16/7 part of the beta-amyloid is cleaved by a protease, such as the alpha-secretase, and the cleaved extracellular domain is secreted out of cell in a type of APP alpha.

APP cleaved by the beta-secretase is divided into a cytoplasmic domain called C99 and N-terminal domain called sAPPβ (secreted form of β-secretase derived APP). C99 again produces 44 kDa beta-amyloid by the gamma-secretase, in which the beta-amyloid has cohesiveness in a small amount, and is beta-amyloid 42 consisting of 42 amino acids that are mainly found in a neurotic plaque. sAPPβ allows secreting about 90 kDa protein outside cell, but its function is not well known (Suh YH, Checler F., Pharmacol Rev 2002; 54:469 -525., Suh YH., J Neurochem 1997; 68: 1781 -91., Selkoe DJ., Physiol Rev 2001; 81: 741 -66).

The features, such as an aggregation and neurotoxicity were confirmed in a sequence of the beta-amyloid. KLVFF (Aβ16-20) that is the middle part of the sequence is important for interacting between the beta-amyloids. In addition, 25-35 part of the beta-amyloid seems to contribute both of an aggregation and neurotoxicity in a peptide (Yang SP. et al., J Neurochem. 2005 Apr; 93(1):118-27.).

Vascular endothelial growth factor (VEGF) is one of important factors for new angiogenesis. VEGF is a homodimeric protein and allows producing five isomers consisting of 121, 145, 165, 189, and 206 amino acids by an alternative splicing. According to the recent studies, it is further reported that VEGF165 has new roles, such as psychotropic and nerve protection factors related to angiogenesis, vascular permeability in vivo, and an endothelial growth (Di Benedetto M., Biochim Biophys Acta. 2008 Apr;1780(4):723-32. Epub 2008 Feb 7.).

VEGF121 and VEGF165 occupy most of VEGF isomers, and also are involved in most of VEGF activity. VEGF165 has higher affinity to VEGF receptor than that of VEGF121, and also high effect on stimulating an endothelial division. All of VEGF isomers have N-terminal receptor-binding domain consisting of 110 amino acids in common. On the contrary, C-terminal is not. VEGF165 has a heparin-binding domain (HBD) consisting of 55 amino acids that can be combined with heparin, and encoded by exon 7 and exon 8. However, VEGF121 does not have HBD so that heparin cannot be combined with it. According to the recent studies, it can be found that VEGF is interacted with the beta-amyloid and affects cytotoxicity of the beta-amyloid. VEGF165 is interacted with the beta-amyloid, but VEGF121 does not have HBD so that it is not interacted with the beta-amyloid. In addition, it is confirmed that the main binding part of VEGF165

that is combined with the beta-amyloid is 25-35 sequence of peptide. As demonstrated in the above sentence, 25-35 part of the beta-amyloid allows the beta-amyloid to have toxicity. VEGF165 protects a cell from neurotoxicity induced by the beta-mayloid and the aggregation of the beta-amyloid (Yang SP. et al., J Neurochem. 2005 Apr;93(1):118-27).

## SUMMARY OF THE INVENTION

The present invention is designed for the above needs, and one object of the present invention is to provide a method for screening a beta-amyloid inhibitor.

Another object of the present invention is to provide a beta-amyloid inhibitor.

In order to achieve the above objects, the present invention provides a method for screening a beta-amyloid inhibitor, including:

a) screening a starting material for screening a protein chip by performing a molecular docking simulation to the whole compound library files;

b) preparing a mixture by mixing the screened starting material with vascular endothelial growth factor (VEGF);

c) adding the mixture to a beta-amyloid-fixed protein chip; and

d) analyzing the degree of binding.

According to an embodiment of the present invention, a search algorithm for the docking calculation preferably uses Alpha Triangle Method, but is not limited thereto.

In addition, according to an embodiment of the present invention, the analysis of the binding degree is preferably performed by reacting with the vascular endothelial growth factor bound to a beta-amlyoid using a primary antibody to be specifically bound to the vascular endothelial growth factor and then using a secondary antibody bound with a fluorescent material that can be bound to the primary antibody, but is not limited thereto. The fluorescent material is preferably at least one selected from the group consisting of Cy3 (Green), Cy5 (Red), FITC (Green), Alexa, BODIPY, Rhodamine, and Q-dot, but is not limited thereto.

In addition, the present invention provides a beta-mayloid inhibitor, including at least one compound selected from the group consisting of the following Chemical Formula 1 and Chemical Formula 2 obtained from the method for screening according to the present invention, and a salt thereof:

[Chemical Formula 1]

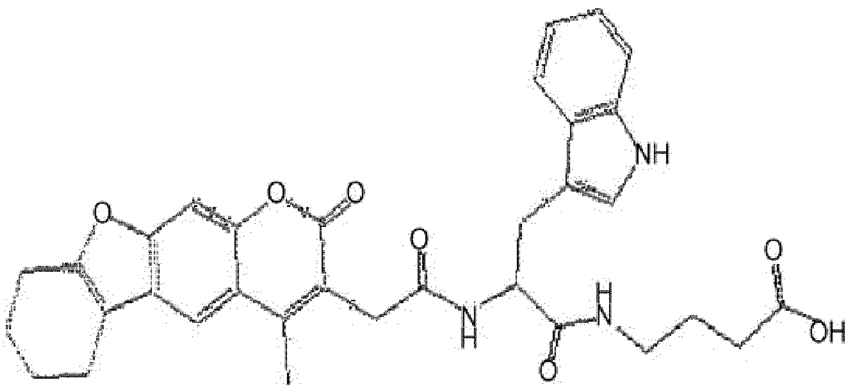

[Chemical Formula 2]

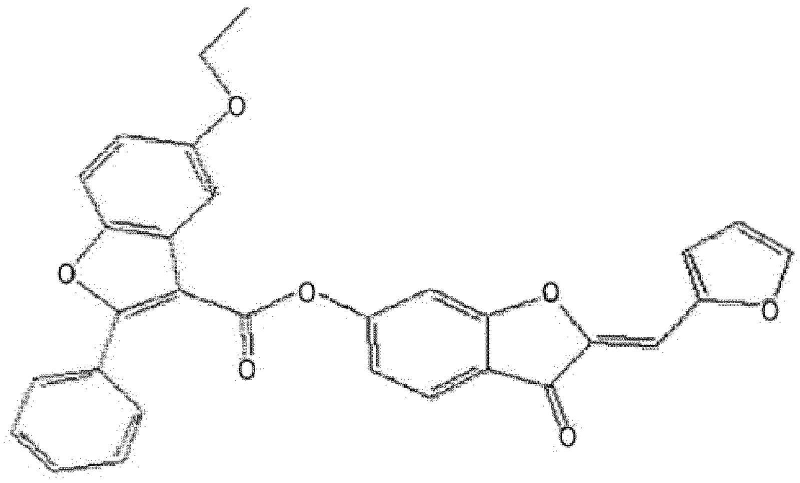

In addition, the present invention provides a beta-amyloid inhibitor containing at least one compound selected from the group consisting of the following Chemical Formula 1 and Chemical Formula 2, and a salt thereof:

[Chemical Formula 1]

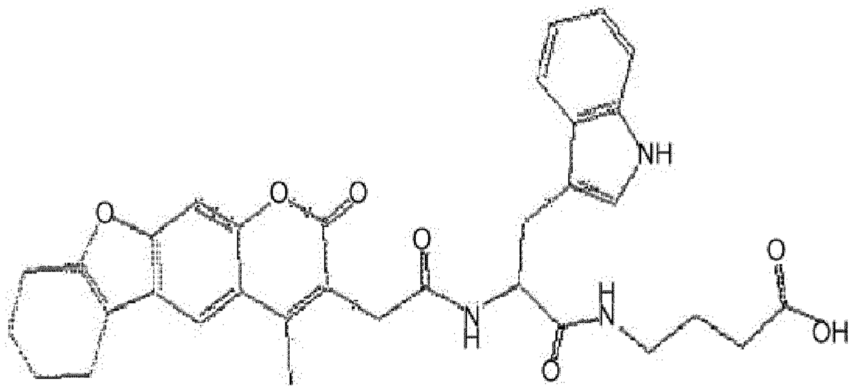

[Chemical Formula 2]

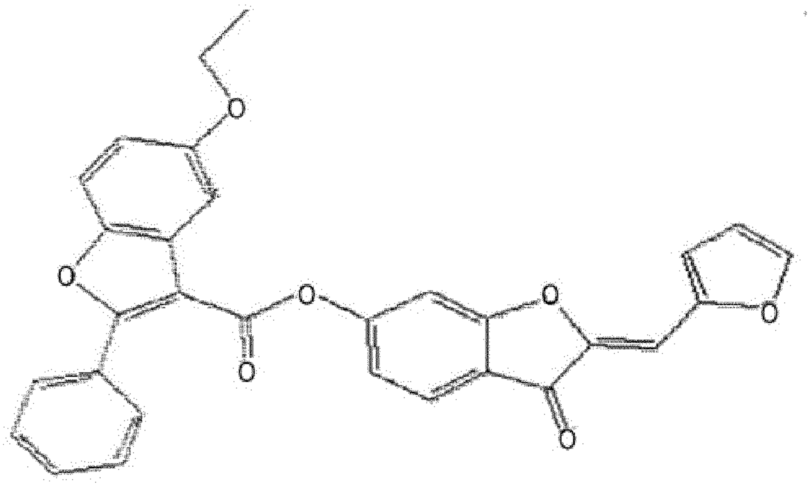

In addition, the present invention provides a composition for treating or preventing Alzheimer's disease, in which the composition includes at least one compound selected from the group consisting of the following Chemical Formula 1 and Chemical Formula 2 obtained from the method for screening according to the present invention, and a salt thereof as an effective component:

[Chemical Formula 1]

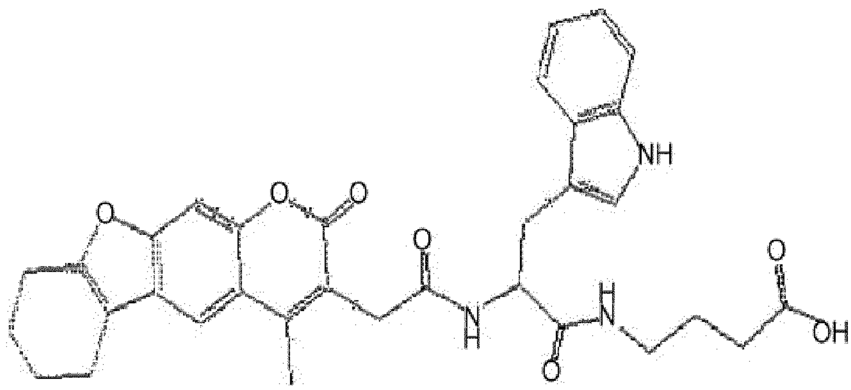

[Chemical Formula 2]

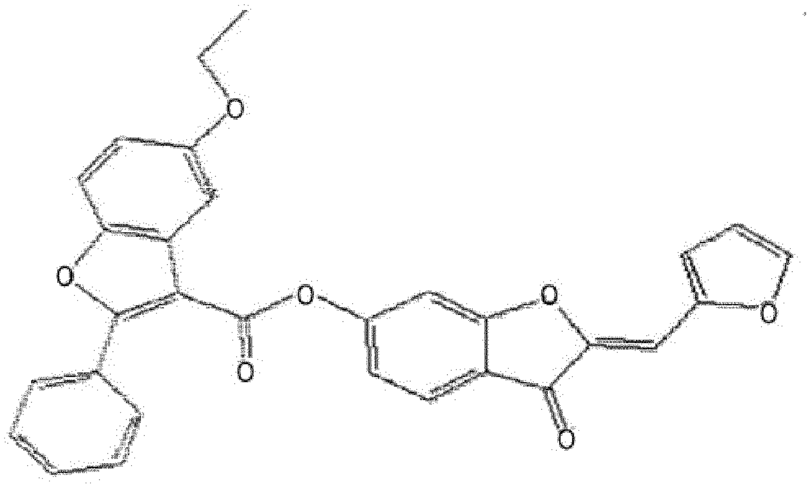

In addition, the present invention provides a composition for treating or preventing Alzheimer's disease, in which the composition includes at least one compound selected from the group consisting of the following Chemical Formula 1 and Chemical Formula 2, or a salt thereof as an effective component:

[Chemical Formula 1]

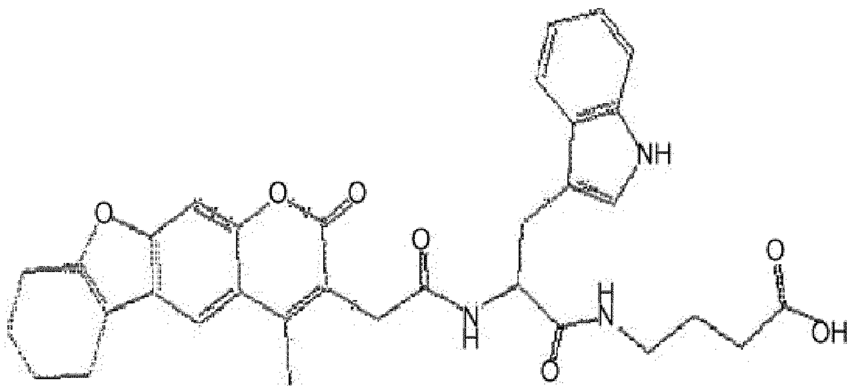

[Chemical Formula 2]

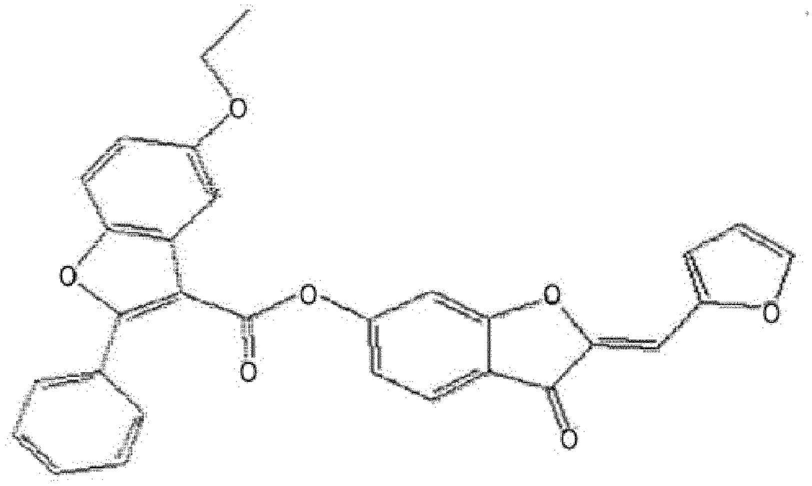

The compound represented by Chemical Formula 1 or Chemical Formula 2, and a pharmaceutical acceptable salt thereof may be used, for example in a form of pharmaceutical drug as a medicine. The pharmaceutical drug may be orally administrated, for example in a type of a tablet, a coated tablet, a sugarcoated pill, soft and hard gelatin capsules, solution, an emulsion, or a suspension. However, also the administration may be performed in a type of suppository through a rectum, or parentally, for example, in a type of injections.

The compound represented by Chemical Formula 1 or Chemical Formula 2 may be processed along with inorganic or organic carrier that is pharmaceutically inert in order to prepare a pharmaceutical drug. For example, lactose, corn starch or derivative thereof, talc, stearic acid or a salt thereof, and the like may be used as a carrier to a tablet, a coated tablet, a sugarcoated pill, and hard gelatine capsules. The carrier suitable for the soft gelatine capsules may be for example, a vegetable oil, wax, fat, semi-solid, liquid polyol, and the like. However, generally the soft gelatin capsule does not need a carrier according to a property of an effective component. The carrier suitable for preparing a solution and syrup may be for example, water, polyol, glycerol, a vegetable oil, and the like. The carrier suitable for a suppository may be for example nature or hydrogenated oil, wax, fat, semi-liquid or liquid polyol, and the like.

Also, the pharmaceutical drug may further include preservatives, a dissolving agent, a stabilizer, a wetting agent, an emulsifier, a sweeting agent, a coloring agent, flavorings, salt for changing an osmotic pressure, a buffer, a masking agent, or an antioxidant. Also, the drug may further include other therapeutic important materials.

One object of the present invention is to provide a drug containing a compound represented by Chemical Formula 1 or Chemical Formula 2 or a pharmaceutical acceptable salt thereof , and a therapeutic inert carrier, as well as a method for preparing them including preparing at least one compound represented by Chemical Formula 1 or Chemical Formula 2, and/or a pharmaceutical acceptable acid additive salt, and some times, at least one of another therapeutic important materials along with at least one of therapeutic inert carriers in a drug administration form.

The compound represented by Chemical Formula 1 or Chemical Formula 1 according to the present invention and also their pharmaceutical acceptable salt are useful to suppress or prevent a disease, for example, Alzheimer's disease.

A dose may be changed within the wide range, and should be adjusted according to individual requirements in an each specific case. In the case of an oral administration, it may be changed to about 0.01 to about 1000 of the compound represented by Chemical Formula 1 or Chemical Formula 2 and the corresponding amount of pharmaceutical acceptable salt as an adult dose per one day.

The dose per one day may be administrated as a single dose or divided doses, and also the upper limitation may be exceeded when the use is directed.

The processes for preparing the drug, filling into a container, and then sealing should be performed under the general antibiotic and aseptic conditions.

The compounds according to the present invention may be prepared by a general method that is known in the art.

Hereinafter, the present invention will be described.

The present invention relates to an inhibitor of an interaction between a beta-amyloid 1-42 peptide and VEGF.

The beta-amyloid 1-42 (Aβ) is most powerful material for developing Alzheimer's disease (AD), and an aqueous amyloid forms β pleated sheet formation to form a fibril so that it is easy to precipitate to have toxicity that is involved in a neural death. At this situation, HBD of VEGF165 is bound to 25-35 part that is a part for involving in toxicity of the beta-amyloid to reduce Aβ toxicity. A similar compound to HBD which inhibits the binding between the beta-amyloid and

VEGF165 may be developed as a raw material for a therapeutic agent and a medicine of Alzheimer's disease. A method for quickly and easily screening ten thousands of compounds is required for screening an inhibitor of the binding between the beta-amyloid and VEGF165. A material screened by using a virtual screening and the protein chip may be developed to an inhibitor of the binding between the beta-amyloid and VEGF165.

A material for inhibiting the binding between the beta-amyloid and VEGF165 helps VEGF165 to act as psychotropic and nerve protection factors through VEGF165 is free from a nerve cell by inhibiting the interaction between VEGF165-beta-amyloid by binding to HBD of VEGF165.

## BRIEF DESCRIPTION OF THE DRAWINGS

The above and other aspects, features and other advantages of the present invention will be more clearly understood from the following detailed description taken in conjunction with the accompanying drawings, in which:

Fig. 1 is a summarized method used for a virtual screening;

Fig. 2 is a result for confirming binding affinities per concentrations according to the interaction between a beta-amyloid 1-42 peptide and VEGF165;

Fig. 3 is a result for inhibiting the interaction between the beta-mayloid 1-42 peptide and VEGF165 from natural products-derived compounds libraries which are screened according to Example 1;

Fig. 4 is $IC_{50}$ (50 % inhibition concentration of maximum inhibition concentration) values as results for experimenting concentration-dependent inhibition abilities of compounds which competitively inhibit the binding between the beta-amyloid and VEGF165 using the compounds obtained during a first screening in Example 3 as an object;

Fig. 5 is names and chemical structures of the materials for inhibiting the interaction between the beta-amyloid and VEGF165;

Fig. 6 is results for confirming the toxicity of the beta-amyloid in PC12 cell and SH-SY5Y cell; and

Fig. 7 is results for confirming the decrease of toxicities of the beta-amyloid by Alzhemed™ and IPS-04001 in SH-SY5Y cell.

## DETAILED DESCRIPTION OF THE PREFERRED EMBODIMENT

Exemplary embodiments of the present invention will now be described in detail with reference to the accompanying drawings.

Hereinafter, the present invention will be described in more detail with reference to non-limited Examples. However, Examples will be only described for illustrating the present invention, but the range of the present invention is not limited to the following Examples.

<u>Example 1: Virtual Screening of Material for Inhibiting Binding between Beta-amyloid and VEGF165</u>

MOE (Molecular Operating Environment) program of Chemical Computing Group and GOLD 4.0.1 program of Cambridge Crystallographic Data Centre (CCDC) were used as software used for a virtual screening. A molecular docking simulation was used as a screening method. The specific method was as follows. Firstly, a first molecular docking simulation was performed to the whole of 40,000 library compounds files. 1KMX structure of Protein Data Bank (PDB) among VEFG models was used as a target receptor protein. A search algorithm for a docking calculation used Alpha Triangle Method to calculate maximum 500,000 structures changes energies per each ligand compound. The method used a docking algorithm by confirming as to whether another triangle of receptor protein was matched to the triangle that was shaped with three points of molecular. LondondG method was used as a scoring method to calculate maximum 10 poses per ligands. The scoring method supported from MOE was divided into three classes, such as, LondondG, AffinitydG, and AlphaHB, and LondondG used for the present calculation was as follows:

$$\Delta G = c + E_{flex} + \sum_{h-bonds} c_{HB} f_{HB} + \sum_{m-lig} c_M f_M + \sum_{atoms\ i} \Delta D_i$$

A rotational/translation entropy change due to a binding, a flexibility energy decrease due to a binding of ligand, hydrogen bond energy, a metal ion ligation, a desolavtion energy difference, and the like were used as a parameter for LondondG function.

A second docking simulation was performed by using 10,000 compounds with a excellent binding affinity that were selected through a result of first docking simulation to 40,000 compounds. The second docking simulation used GOLD program to perform an arithmetic operation using Slow Option. GOLD program supported three options, such

as, GoldScore, ChemScore, and ASPScore, and the second docking simulation used GoldScore. 140 compounds with most excellent LondondG value that was a resultant score for minimizing energy and docking simulation to 10,000 compounds were selected to use as a starting material for screening a protein chip (see Table 1). Table 1 shown serial numbers of 140 compounds with most excellent binding affinity selected from the result of virtual screening.

[Table 1]

| No. | ID | No. | ID | No. | ID | No. | ID | No. | ID | No. | ID | No. | ID |
|---|---|---|---|---|---|---|---|---|---|---|---|---|---|
| 1 | 39787 | 21 | 50933 | 41 | 21243 | 61 | 69740 | 61 | 60844 | 101 | 42910 | 121 | 38766 |
| 2 | 61741 | 22 | 49565 | 42 | 37322 | 62 | 69522 | 82 | 65980 | 102 | 40269 | 122 | 39720 |
| 3 | 50368 | 23 | 62003 | 43 | 31455 | 63 | 6618 | 83 | 37589 | 103 | 54134 | 123 | 37799 |
| 4 | 370 | 24 | 38344 | 44 | 28186 | 64 | 65595 | 84 | 65539 | 104 | 66060 | 124 | 50049 |
| 5 | 1115 | 25 | 37479 | 45 | 68021 | 65 | 49816. | 85 | 9796 | 105 | 51288 | 125 | 31168 |
| 6 | 50337 | 26 | 11871 | 46 | 38414 | 66 | 62241 | 86 | 67794 | 106 | 52867 | 126 | 61260 |
| 7 | 65681 | 27 | 39862 | 47 | 47309 | 67 | 42520 | 87 | 67720 | 107 | 37897 | 127 | 37615 |
| 8 | 2197 | 28 | 44053 | 48 | 67525 | 68 | 41834 | 88 | 6218 | 108 | 1280 | 128 | 65841 |
| 9 | 68318 | 29 | 65760 | 49 | 40256. | 69 | 60353 | 89 | 67464 | 109 | 41400 | 129 | 18414 |
| 10 | 59803 | 30 | 70301 | 50 | 39912 | 70 | 42258 | 90 | 64496 | 110 | 68153 | 130 | 68191 |
| 11 | 11801 | 31 | 43520 | 51 | 68314 | 71 | 68182 | 91 | 35103 | 111 | 10487 | 131 | 66301 |
| 12 | 44662 | 32 | 61095 | 52 | 66940 | 72 | 11989 | 92 | 40362 | 112 | 40071 | 132 | 65123 |
| 13 | 66500 | 33 | 41845 | 53 | 38415 | 73 | 55735 | 93 | 66410 | 113 | 40528 | 133 | 55891 |
| 14 | 63891 | 34 | 66085 | 54 | 69868 | 74 | 4156 | 94 | 65440 | 114 | 61365 | 134 | 52047 |
| 15 | 59013 | 35 | 67797 | 55 | 39957 | 75 | 67961 | 95 | 5087 | 115 | 51230 | 135 | 21725 |
| 16 | 57714 | 36 | 39663 | 56 | 39075 | 76 | 68161 | 96 | 52620 | 116 | 20842 | 136 | 70254 |
| 17 | 37987 | 37 | 40948 | 57 | 69319 | 77 | 39693 | 97 | 3884 | 117 | 40996 | 137 | 38166 |
| 18 | 56949 | 38 | 40632 | 58 | 38245 | 78 | 56474 | 98 | 67312 | 118 | 34042 | 138 | 65730 |
| 19 | 63766 | 39 | 69183 | 59 | 38352 | 79 | 37996 | 99 | 36531 | 119 | 49921 | 139 | 41881 |
| 20 | 47947 | 40 | 38006 | 60 | 68274 | 80 | 39922 | 100 | 38864 | 120 | 36640 | 140 | 61083 |

Table 1 is a summarized table of methods used for virtual screening.

Example 2: Fixation of Beta-amyloid to Substrate

ProteoChip™ (Proteogen Inc., Seoul, Korea) was used as a substrate to fix a protein. A sheet paper was adhered on the substrate to prepare Well-Chip. A beta-amyloid (Bachem AG, Bubendorf, Switzerland) was diluted with a phosphate-buffered saline (PBS) containing 30 % glycerol solution to be 50μg/ml; then added to each well; reacted at 30 °C in a humidity incubator, overnight; the beta-amyloid remained after combining was washed with a phosphate-buffered saline containing 0.05 % tween-20 (0.05% PBST); dried with a nitrogen gas; and then a high-speed-high throughput screening was performed.

<u>Example 3: High-Speed-High-Throughput Screening for Material of inhibiting Interaction between Beta-amyloid and VEGF165 from Library</u>

3-1) Interaction of Beta-amyloid and VEGF165

In order to investigate an interaction of a beta-amyloid and VEGF165, the beta-amyloid-tagged protein chip prepared in Example 2 was blocked with 3 % BSA for 2 hours; then washed with a washing solution (0.05 % PBST) twice; and then dried with a nitrogen gas. And then, VEGF165 (R&D System, Inc., Minneapolis, MN USA) with the concentration range of 500 μg/ml to 3.9 μg/ml was diluted with a phosphate-buffered saline (PBS) containing 30 glycerol solution and then added to each of wells of the beta-amyloid microarray; reacted at 30 °C in a humidity incubator for 1 hour; unbound VEGF165 to the beta-amyloid was washed with the washing solution twice; and then dried using a nitrogen gas. And then, it was reacted with VEGF165 bound to the beta-amyloid using VEGF165 antibody (Primary Antibody) to be specifically bound to VEGF165 at 30 °C in a humidity incubator for 1 hour; unbound primary antibody was washed

with the washing solution twice; and then dried with a nitrogen gas. And then, the antibody (Secondary Antibody) bound with Cy5 fluorescent material (Cy-5; Amersham Parmacia Biotech, Uppsala Sweden) that can be bound to the primary antibody was reacted at 30°C in humidity incubator for 30 minutes. Unbound secondary antibody was washed with the washing solution twice; dried with the nitrogen gas; and then scanned with a fluorescent scanner.

Fig 2 is a fluorescent scan image (Left part of Fig. 2) showing the interaction between a beta-amyloid 1-42 peptide and VEGF165 and a graph (Right part of Fig. 2) showing a dose-response curve of the interaction between the beta-amyloid 1-42 peptide and VEGF165. Specifically, Fig. 2 is a graph showing a relative fluorescence intensity of VEGF165 bound to the beta-amyloid and a primary antibody bound to VEGF165 by measuring the relation between the logs of the secondary antibody concentration marked with Cy-5 fluorescence.

As shown in Fig. 2, it could be found that VEGF165 was bound to the beta-amyloid and then bound to the primary antibody, and then the primary antibody well reacts with Cy5-marked secondary antibody. Among those, VEGF165 was started to saturate at the concentration of at least about 125 μg/ml. For this reason, it could be known that the beta-amyloid 1-42 peptide-tagged chip was useful and suitable for screening an inhibitor of the binding between the beta-amyloid 1-42 peptide and VEGF165.

3-2) Preparation of Mixed Solution of VEGF165 and Library

A mixed solution was prepared by mixing VEGF165 (50 μg/Me) and 130 compounds (50 μM) derived from a natural material obtained in Example 1 in order to experiment the inhibition of the binding between the beta-amyloid and VEGF165. It was diluted with the phosphate-buffered saline (PBS) containing 30 % glycerol solution to use.

3-3) First Screening of Inhibitor for inhibiting Binding between Beta-amyloid and VEGF165

The beta-amyloid-tagged protein chip prepared in Example 2 was blocked with 3 % BSA for 2 hours; then washed with 0.05 % PBST twice; and then dried with a nitrogen gas. And then, the mixed solution containing each of 130 libraries prepared in Example 3-2 (a single concentration of 150 μM) and VEGF165 was added to each of wells; and then reacted at 30 °C in a humidity incubator for 1 hour. It was washed with the washing solution; then dried using a nitrogen gas; a primary antibody was added to each of wells; then reacted at 30 °C in a humidity incubator for 30 minutes; washed with the washing solution; and then dried with the nitrogen gas to measure an inhibition ability of library by analyzing the binding degree with the relative fluorescence intensity using a fluorescence laser scanner. As a result, it could be verified that the compounds effectively inhibited the binding reaction of the beta-amyloid 1-42 peptide-VEGF165 by showing relatively low fluorescence intensity in a great number of libraries (see Fig. 3).

Fig. 3 is the results for screening the compounds for inhibiting the interaction of the beta-amyloid 1-42 peptide and VEGF165 from the library in a protein chip system.

For the above experiment, the mixed solution without the library was used as a positive control and the mixed solution with a heparin was used as a negative control. The heparin is bound to HBD of VEGF165 to inhibit VEGF165 to bind to the beta-amyloid.

The fluorescence intensity in Fig. 3 expresses rainbow colors. Originally, the result expresses single color, such as blue or red, but in the case of the above experiment, the software of the device gives the color changed according to the fluorescence intensity because the fluorescence intensity was not easily distinguished if the color was single.

Generally, the fluorescence intensity appears from the strongest fluorescence intensity in the order of white, red, orange, yellow, green, and blue. From the result of Fig. 3, it could be found that the color was white or red when reacting only with VEFG165 so that the binding between the beta-amyloid 1-42 peptide and VEGF165 was presented. When experimenting with the heparin, the interaction between the beta-amyloid 1-42 peptide and VEGF165 was inhibited so that the beta-amyloid 1-42 peptide and VEGF165 was not bound thereby appearing blue that means the lowest fluorescence intensity.

Some compounds of libraries inhibit the interaction between the beta-amyloid 1-42 peptide and VEGF165 so that the beta-amyloid 1-42 peptide and VEGF165 were not bound thereby appearing blue or green that means the lowest fluorescence intensity. Therefore, it was verified that the specific compound was the material that inhibits the binding between the beta-amyloid 1-42 peptide and VEGF164 (see Fig. 3).

3-4) Second Screening of Inhibitor for inhibiting Binding between Beta-amyloid and VEGF165

$IC_{50}$ values for inhibiting the binding between VEGF165 in the concentration of 50 μg/ml and the beta-amyloid fixed in the concentration of 50 ug/ml were obtained by treating the inhibitors found from the first screening in Example 3-3 using the method used in Example 3-3, in which the inhibitors were used by diluting two times from 100 μM to 1 μM per concentration (see Fig. 4). The results for two types of inhibitors that had most effective inhibition activity among those were shown (see Fig. 5).

<u>Example 4: Observation of Biological Activity for Materials Screened Using Protein Chip</u>

4-1) Preparation of PC12 Cell and SH-SY5Y Cell

PC12 Cell (Korean Cell Line Bank, Seoul, Korea) was maintained in the mixed solution of RPM11640 (Welgene, Daegu, Korea) containing 1X Antibiotic-Antimycotic (GIBCO, N.Y., USA) and 10% FBS (Fetal Bovine Serum) (Welgene, Daegu, Korea). SH-SY5Y cell (Korean Cell Line Bank, Seoul, Korea) was maintained in the mixed solution of DMED/F12 (Welgene, Daegu, Korea) containing 1X Antibiotic-Antimycotic (GIBCO, N.Y., USA) and 10% FBS (Fetal Bovine Serum)

(Welgene, Daegu, Korea). The PC12 and SH-SY5Y culture cells were maintained under the conditions of 100 % humidity and 37 °C in the environment for supplying gas components with further 5 % carbon dioxide.

4-2) MTT assay

It was performed according to a MTT [(3-(4,5-dimethylthiazol-2yl-2,5-diphenyl-2H-tetrazoilum bromide] experiment protocol. MTT was dissolved in PBS to be 5mg/m! and then stored at 4°C to use. After completing the reaction, 10 μℓ of 5 mg/ml MTT solution was added to 100 culture mixed solution of each well and then reacted for 3 hours. The supernatant of each well was discarded, and then a chromophoric reactant (Formazancrystal) was dissolved in 100 μℓ of DMSO; and then an absorbance was measured using ELISA reader (595 nm).

4-3) Cytotoxicity of Beta-amyloid

The PC12 cells were added to each of wells of Poly-D-lysine-coated 96-well tissue culture plate (Corning, MA , USA) to be the cell concentration of $2x10^4$ per well. And then, after maintaining for 24 hours, the beta-amyloid was added to each well per concentrations along with the mixed solution containing 10 % FBS, and then reacted for 24 hours to perform MTT assay.

4-4) Inhibition of Cytotoxicity of Beta-amyloid by Screened Material

The SH-SY5Y cells were added to 96-well tissue culture plate to be the cell concentration of $1x10^4$ per well. And then, after maintaining for 24 hours, it was starved with the culture mixed solution containing 2 % FBS for 4 hours; the beta-amyloid (10 μM) and the screened material (1/2 diluted in 20 mM) were added to each well along with the culture mixed solution containing 2 % FBS; and then reacted for 24 hours to perform MTT assay.

Fig. 6 is results for confirming cytotoxicity by the beta-amyloid in SH-SY5Y cell and PC12 cell. It could be confirmed that a survival rate of cell was decreased in proportion as the concentration increase of beta-amyloid 1-42 in PC12 cell and SH-SY5Y cell, and also a survival rate of cell was not largely decreased as compared with that of the forward-synthesized beta-amyloid in proportion as the concentration increase of reverse-synthesized beta-amyloid 42-1 in SH-SY5Y. It was verified that the cytotoxicity was increased by the forward-synthesized beta-amyloid.

Fig. 7 is results for confirming the cytotoxicity decrease of the beta-amyloid by Alzhemed™ and a candidate of inhibitor (IPS-04001) in SH-SY5Y cell. It could be confirmed that a survival rate of cell was confirmed by treating the candidate of inhibitor and Alzhemed™ (1/2 diluted in 20 μM) per concentrations to 10 μM beta-amyloid in SH-SY5Y cell so that the survival rate of cell was increased by the candidate of inhibitor and Alzhemed™. The survival rate was increased to about 30 % at more than 5 μM when using the standard of 10 μM beta-amyloid in Alzhemed™ used as a control, and increased to about 20~25 % at more than 5 μM in the candidate of inhibitor (IPS-04001). And also, it could be found that the survival rate of SH-SY5Y cells was increased to at least 10 % at more than 1.25 μM in the candidate of inhibitor (IPS-04001). Overall, the candidate of inhibitor (IPS-04001) showed the survival rate having about 5 % lower as compared with that of Alzhemed™, but for the toxicity of the beta-amyloid, it was shown the similar survival rates of cells in general.

While the present invention has been shown and described in connection with the exemplary embodiments, it will be apparent to those skilled in the art that modifications and variations can be made without departing from the spirit and scope of the invention as defined by the appended claims.

## Claims

**1.** A method for screening a beta-amyloid inhibitor, comprising:

a) screening a starting material for screening a protein chip by performing a molecular docking simulation to the whole compound library files;
b) preparing a mixture by mixing the screened starting material with vascular endothelial growth factor (VEGF);
c) adding the mixture to a beta-amyloid-fixed protein chip; and
d) analyzing the degree of binding.

**2.** The method of claim 1, wherein a search algorithm for the docking simulation uses Alpha Triangle Method.

**3.** The method of claim 1, wherein an analysis of the binding degree is performed by reacting with the vascular endothelial growth factor bound to the beta-amyloid using a primary antibody to be specifically bound to the vascular endothelial growth factor and then using a secondary antibody bound with a fluorescent material that can be bound to the primary antibody.

**4.** The method of claim 3, wherein the fluorescent material is at least one fluorescent material selected from the group consisting of Cy3 (Green), Cy5 (Red), FITC (Green), Alexa, BODIPY, Rhodamine, and Q-dot.

5. A beta-amyloid inhibitor comprising at least one compound selected from the group consisting of the following Chemical Formula 1 and Chemical Formula 2 obtained from the method for screening according to any one of claims 1 to 3, and a salt thereof:

[Chemical Formula 1]

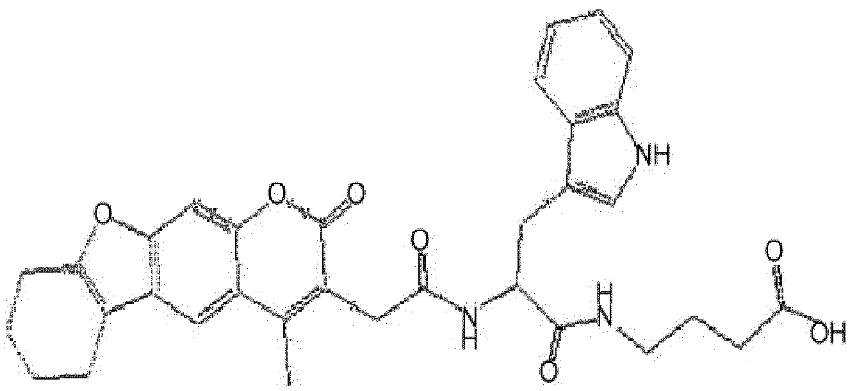

[Chemical Formula 2]

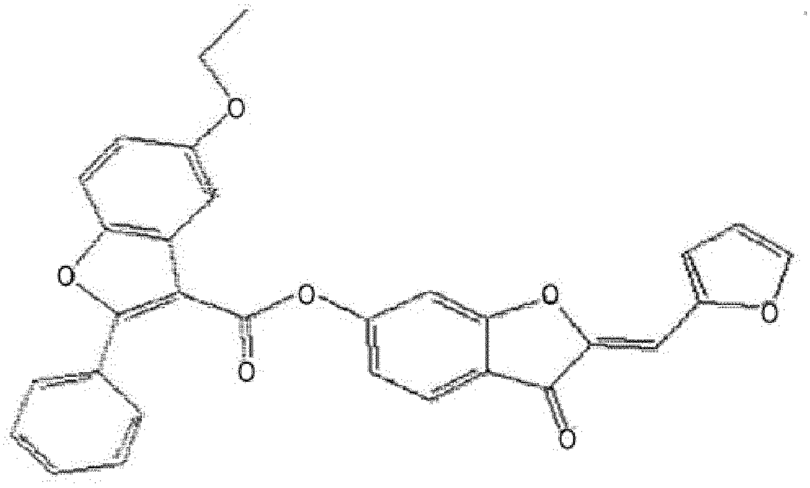

6. A beta-amyloid inhibitor comprising at least one compound selected from the group consisting of the following Chemical Formula 1 and Chemical Formula 2, and a salt thereof:

[Chemical Formula 1]

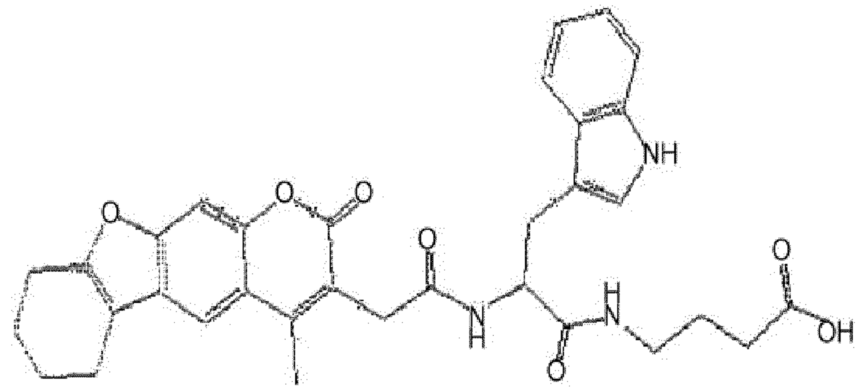

[Chemical Formula 2]

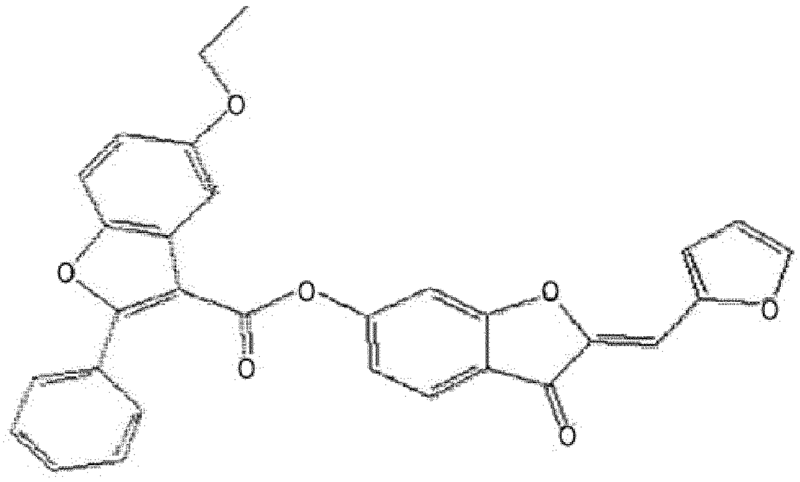

7. A composition for treating or preventing Alzheimer's disease, comprising at least one compound selected from the group consisting of the following Chemical Formula 1 and Chemical Formula 2 obtained from the method for screening according to any one of claims 1 to 3, and a salt thereof as an effective component:

[Chemical Formula 1]

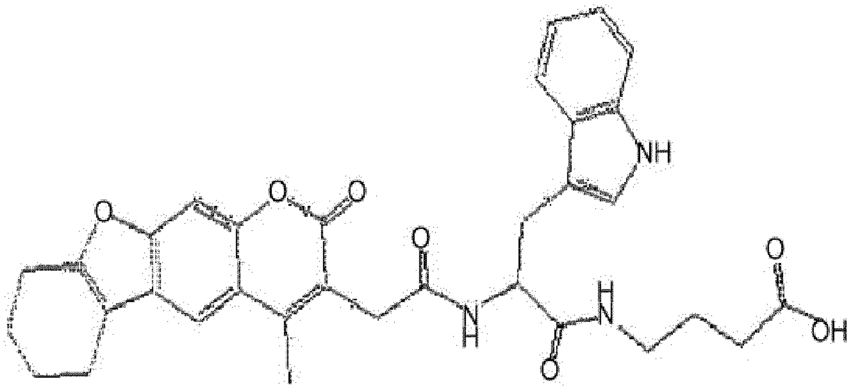

[Chemical Formula 2]

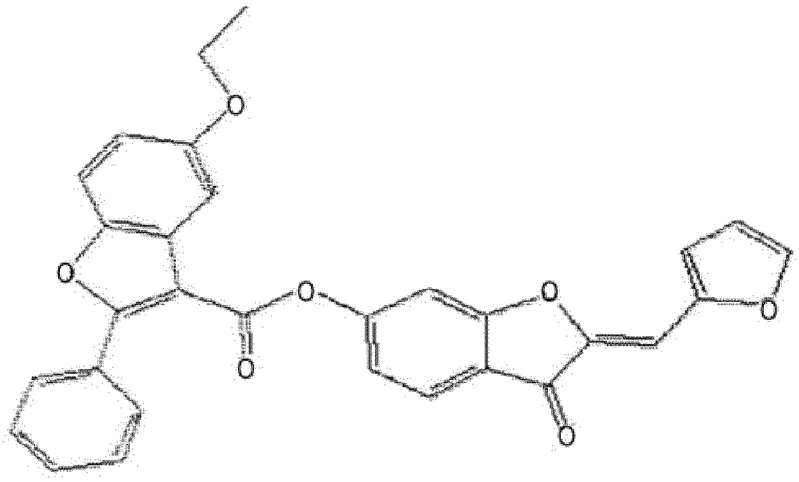

**8.** A composition for treating or preventing Alzheimer's disease, comprising at least one compound selected from the group consisting of the following Chemical Formula 1 and Chemical Formula 2, and a salt thereof as an effective component:

[Chemical Formula 1]

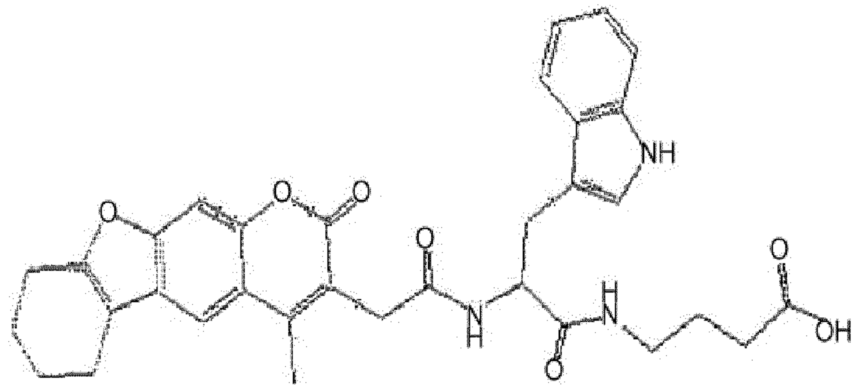

[Chemical Formula 2]

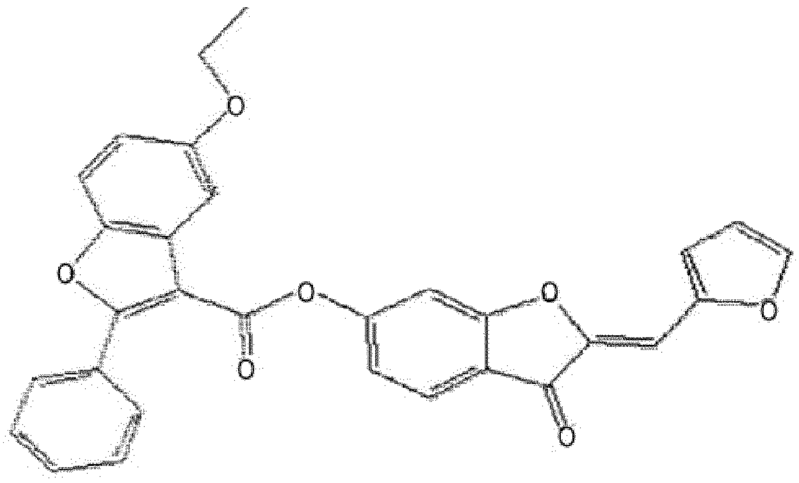

[FIG. 1]

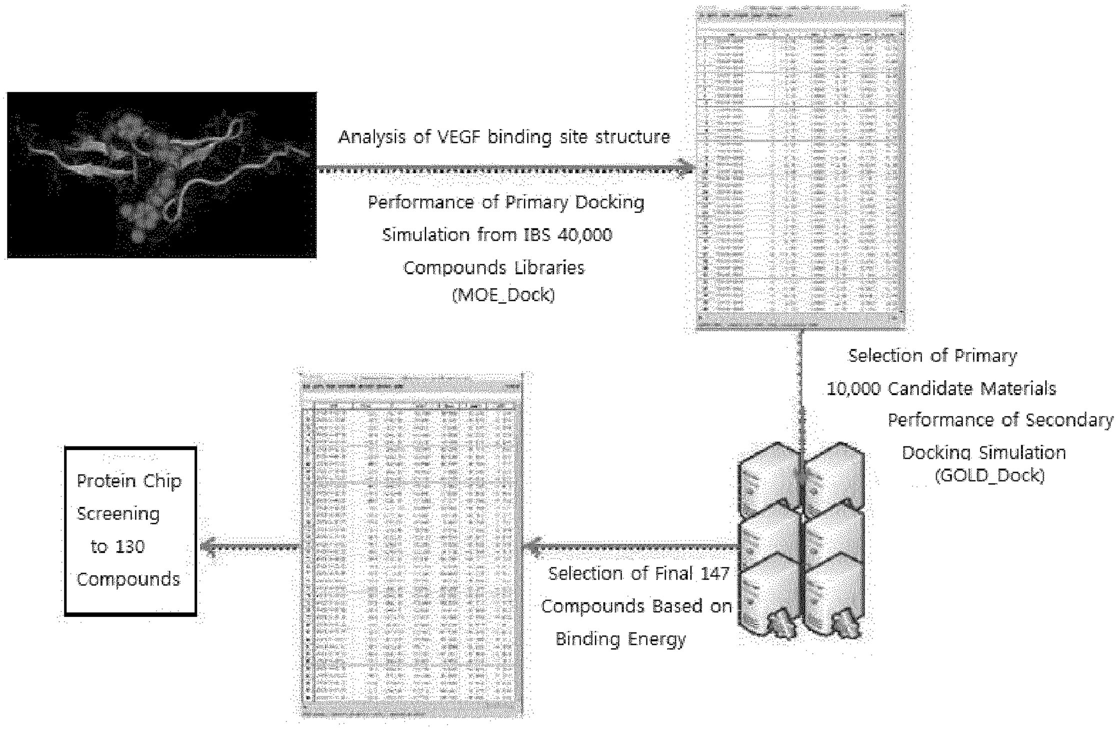

Analysis of VEGF binding site structure
Performance of Primary Docking Simulation from IBS 40,000 Compounds Libraries (MOE_Dock)
Selection of Primary 10,000 Candidate Materials
Performance of Secondary Docking Simulation (GOLD_Dock)
Selection of Final 147 Compounds Based on Binding Energy
Protein Chip Screening to 130 Compounds


[FIG. 2]

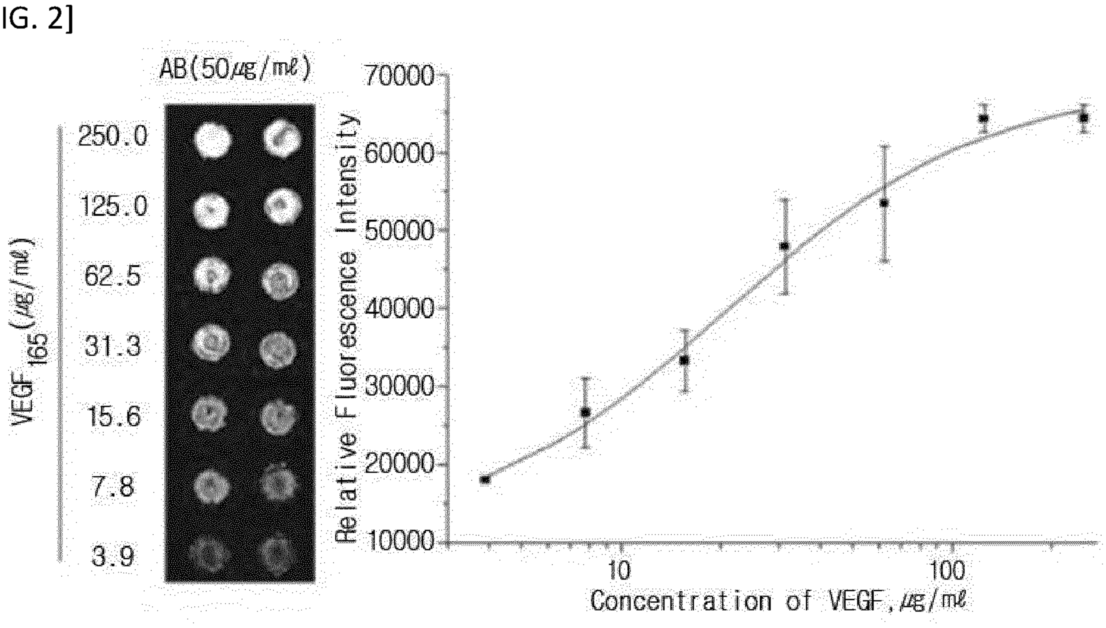

AB(50㎍/㎖)
$VEGF_{165}$(㎍/㎖)
250.0
125.0
62.5
31.3
15.6
7.8
3.9
Relative Fluorescence Intensity
70000
60000
50000
40000
30000
20000
10000
10
100
Concentration of VEGF, ㎍/㎖

[F[[FIG. 3]

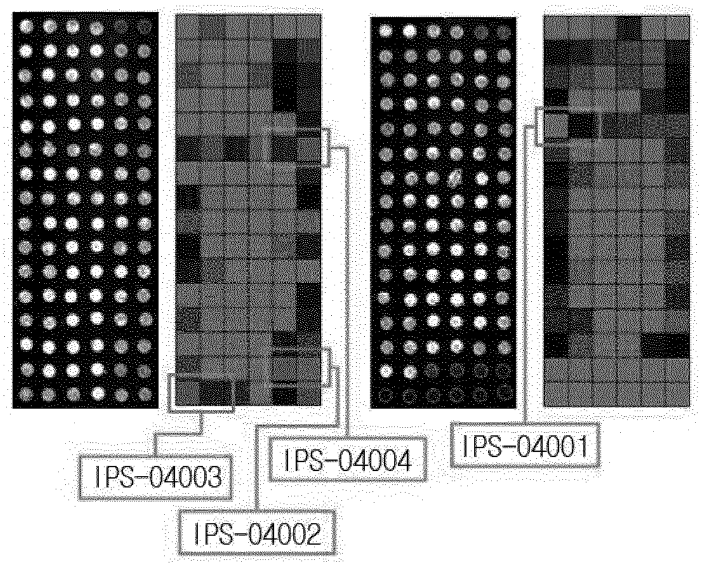
IPS-04003
IPS-04004
IPS-04001
IPS-04002

[FIG. 4]

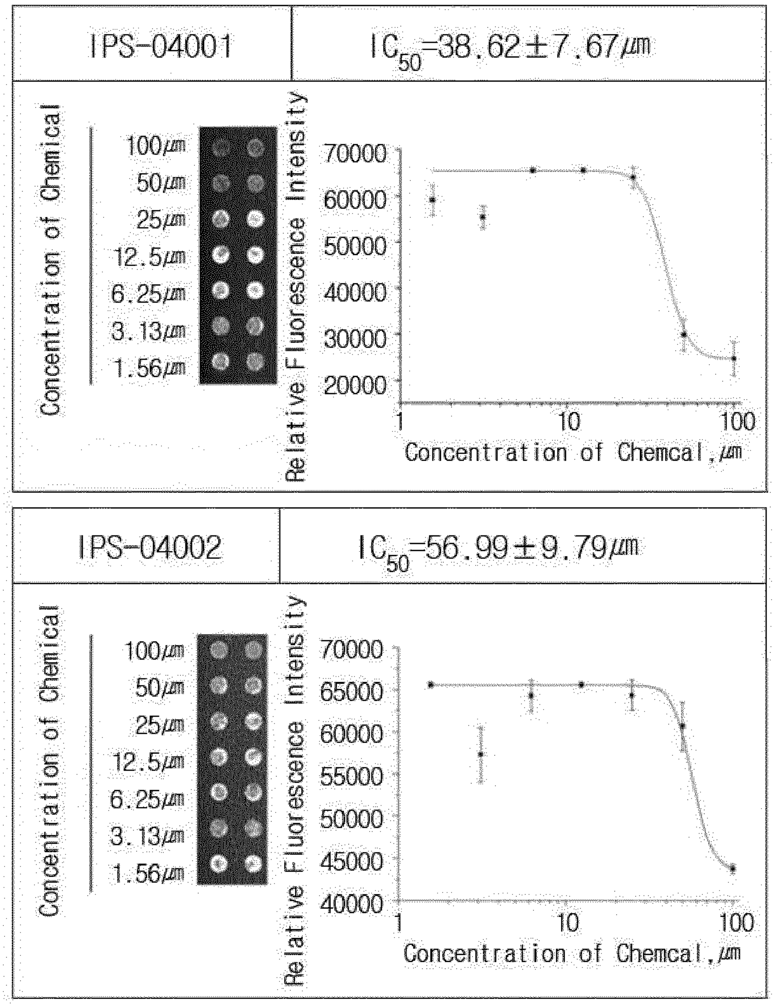
IPS-04001
$IC_{50}$=38.62±7.67㎛
Concentration of Chemical
100㎛
50㎛
25㎛
12.5㎛
6.25㎛
3.13㎛
1.56㎛
Relative Fluorescence Intensity
70000
60000
50000
40000
30000
20000
1
10
100
Concentration of Chemcal,㎛
IPS-04002
$IC_{50}$=56.99±9.79㎛
Concentration of Chemical
100㎛
50㎛
25㎛
12.5㎛
6.25㎛
3.13㎛
1.56㎛
Relative Fluorescence Intensity
70000
65000
60000
55000
50000
45000
40000
1
10
100
Concentration of Chemcal,㎛

[F[[FIG. 5]

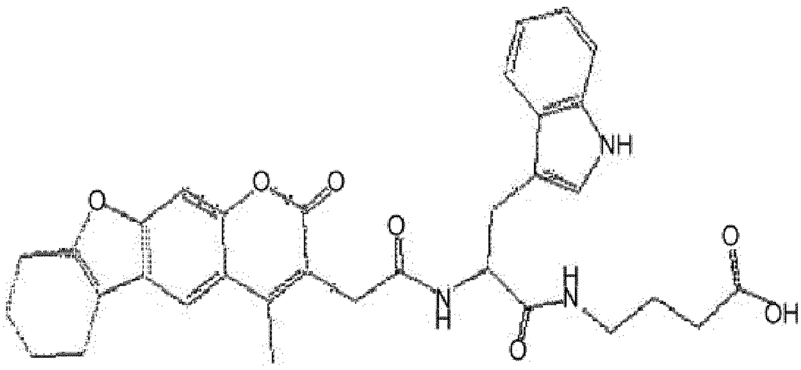

(IPS-04001) : 4-{3-(1*H*-Indol-3-y1)-2-[2-(6-methyl-8-oxo-1,2,3,4-tetrahydro-8*H*-9,11-dioxa-benzo[*b*]flioren-7-y1)-acetylamino]-propionylamino}-butyric acid

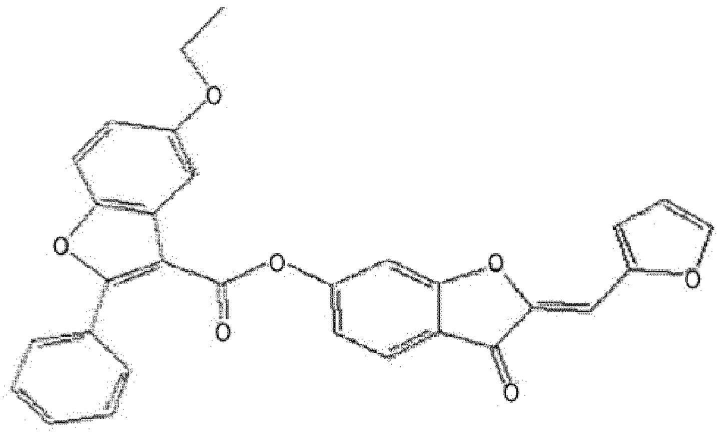

(IPS-04002) : 5-Ethoxy-2-phenyl-benzofuran-3-carboxylic acid 2-furan-2-y1methylene-3-oxo-2,3-dihydro-benzofyran-6-y1ester

[[FIG. 6]

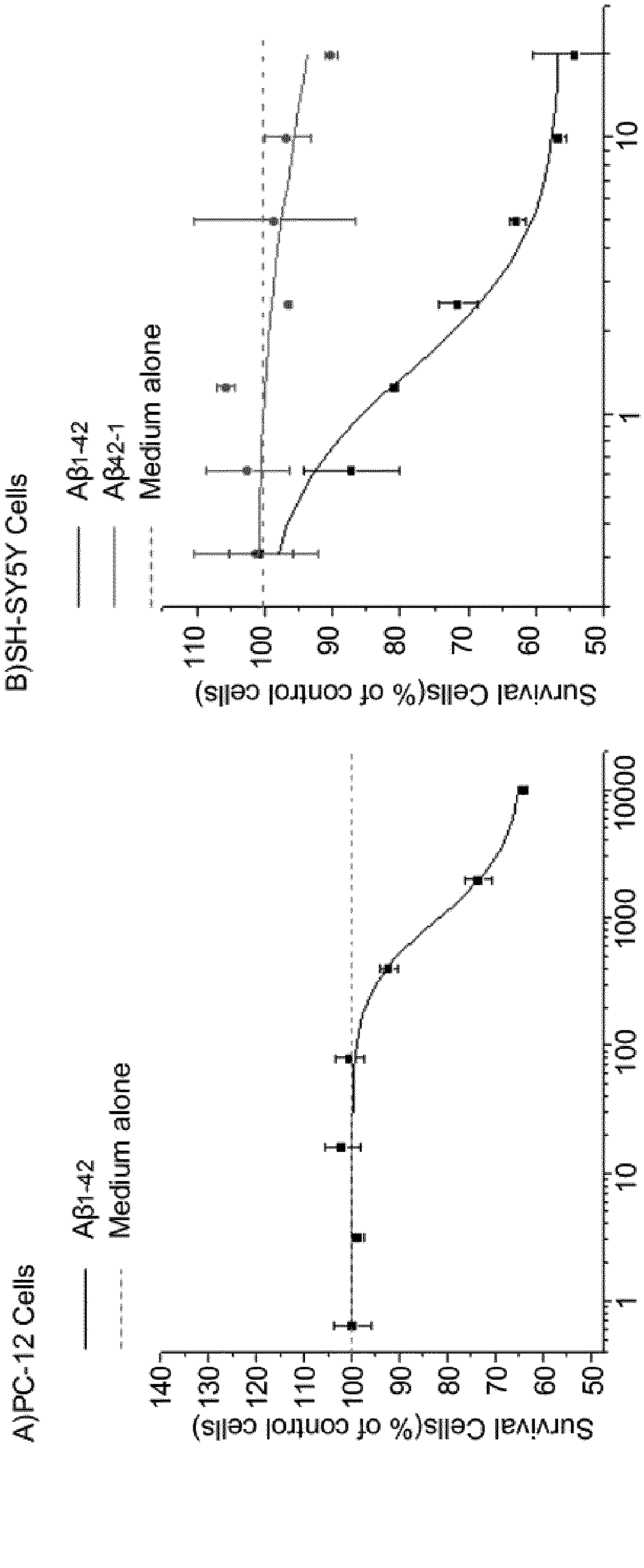
A)PC-12 Cells
Aβ1-42
Medium alone
Survival Cells(% of control cells)
140
130
120
110
100
90
80
70
60
50
1
10
100
1000
10000
beta-Amyloid Concentrations, nM
B)SH-SY5Y Cells
Aβ1-42
Aβ42-1
Medium alone
Survival Cells(% of control cells)
110
100
90
80
70
60
50
1
10
beta-Amyloid Concentrations, μM

[[FIG. 7]

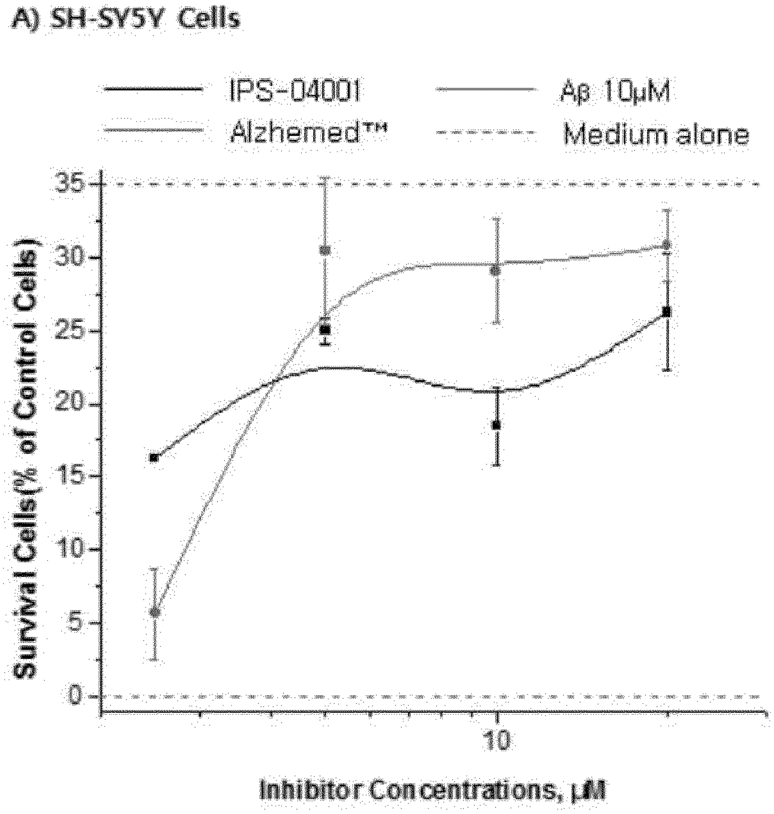
A) SH-SY5Y Cells
IPS-04001
Aβ 10μM
Alzhemed™
Medium alone
Survival Cells(% of Control Cells)
35
30
25
20
15
10
5
0
10
Inhibitor Concentrations, μM

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020090054085

### Non-patent literature cited in the description


- **Parihar MS, Hemnani T.** *J Clin Neurosci.,* June 2004, vol. 11 (5), 456-67
- **Selkoe DJ.** *Nature,* 24 June 1999, vol. 399 (6738), A23-31
- **Suh YH ; Checler F.** *Pharmacol Rev,* 2002, vol. 54, 469-525
- **Suh YH.** *J Neurochem,* 1997, vol. 68, 1781-91
- **Selkoe DJ.** *Physiol Rev,* 2001, vol. 81, 741-66
- **Yang SP. et al.** *J Neurochem.,* April 2005, vol. 93 (1), 118-27
- **Di Benedetto M.** *Biochim Biophys Acta,* 07 February 2008, vol. 1780 (4), 723-32